# FASCICULE DE BREVET EUROPEEN

(11) **EP 3 120 832 B1**
(45) Date de publication et mention de la délivrance du brevet: **09.05.2018**
(21) Numéro de dépôt: 16180649.2
(22) Date de dépôt: 21.07.2016
(51) Int. Cl.: A61K 8/97, G01N 33/50, G01N 33/00, A61K 8/37, A61Q 19/00

(54) **UTILISATION DE L'ACIDE LÉONTOPODIQUE COMME MARQUEUR D'UN EXTRAIT DE RACINE D'IMMORTELLE**
VERWENDUNG DER LEONTOPODISCHEN SÄURE ALS MARKER EINES WURZELEXTRAKTS VON STROHBLUMEN
USE OF LEONTOPODIC ACID AS A MARKER OF AN IMMORTELLE ROOT EXTRACT

(30) Priorité: 23.07.2015 FR 1557017
(43) Date de publication de la demande: 25.01.2017
(73) Titulaire: LABORATOIRES M&L, 04100 Manosque (FR)
(72) Inventeur: ROUQUET, Virginie, 04100 Manosque (FR); PORTES, Pascal, 13540 Puyricard (FR); COMBES, Charline, 04100 Manosque (FR)
(74) Mandataire: Novagraaf Technologies

(56) Documents cités:
- EP-B1- 1 340 071
- WO-A2-2012/153064
- SCHWAIGER S ET AL: "Leontopodic acid-a novel highly substituted glucaric acid derivative from Edelweiss (Leontopodium alpinum Cass.) and its antioxidative and DNA protecting properties", TETRAHEDRON, ELSEVIER SCIENCE PUBLISHERS, AMSTERDAM, NL, vol. 61, no. 19, 9 mai 2005 (2005-05-09), pages 4621-4630, XP027862041, ISSN: 0040-4020 [extrait le 2005-05-09]
- Serhat Sezai Cicek ET AL: "Caffeoyl-D-Glucaric Acid Derivatives in the Genus Gnaphalium (Asteraceae: Gnaphalieae)", Rec. Nat. Prod., 1 janvier 2012 (2012-01-01), pages 311-315, XP055271095, Extrait de l'Internet: URL:http://www.acgpubs.org/RNP/2012/Volume %206/Issue%201/44-RNP-1102-492.pdf [extrait le 2016-05-09]

## Description

### Domaine technique

La présente invention se rapporte à un procédé d'identification de l'origine d'un extrait de racines tel que défini dans les revendications. La présente invention se rapporte également à l'utilisation de l'acide léontopodique comme marqueur de la présence d'un extrait de racines d'immortelle dans un échantillon. La présente invention trouve une application notamment dans les domaines analytique, cosmétique et dermatologique.

### Etat de la technique

Depuis de nombreuses années, les compositions cosmétiques pour l'hygiène capillaire et/ou corporelle et/ou pour le soin de la peau comprennent de nombreux extraits/ingrédients.

Il existe actuellement un intérêt croissant pour l'identification et l'utilisation d'extraits issus de plantes pour leur propriété cosmétique.

En effet, ces extraits sont des extraits naturels considérés comme bénéfiques tant au niveau efficacité cosmétique mais également au niveau environnemental.

Toutefois, de part la diversification des extraits ainsi que la multiplicité de leur source, leur identification ainsi que leur caractérisation reste un besoin important.

De plus, il existe de nombreuses variétés de plantes qui peuvent être désignées sous un même nom vernaculaire. Il existe donc un réel besoin de trouver un moyen et/ou des marqueurs permettant d'identifier différents extraits, par exemple de par leur origine végétale et/ou la partie de la plante dont ils sont issus.

EP1340071 décrit un procédé de détection et identification de constituants d'extraits de plantes ou autres au moyen de techniques chromatographiques.

### Description de l'invention

La présente invention a précisément pour but de répondre à ces nombreux besoins en fournissant un procédé d'identification de l'origine d'un extrait de racine tel que défini dans les revendications. En particulier, le procédé de la présente invention permet avantageusement d'identifier un extrait de racine d'immortelle.

La présente invention a donc pour objet un procédé d'identification de l'origine d'un extrait de racine, ledit procédé comprenant une étape de recherche dans ledit extrait de racines d'acide léontopodique, la présence d'acide léontopodique dans ledit extrait indiquant qu'il s'agit d'un extrait de racines d'immortelle.

En particulier, les inventeurs ont démontré de manière surprenante que les extraits de racines d'immortelle comprennent une concentration d'acide léontopodique significative contrairement aux extraits issus de toute autre partie de la plante, en particulier de l'immortelle.

En outre, il est connu dans l'état de la technique que l'acide léontopodique est présent uniquement dans les parties aériennes de l'edelweiss, Leontopodium alpinum Cass. Asteraceae. Aussi, de manière surprenante et inattendue, les inventeurs ont identifié la présence d'acide léontopodique dans des extraits de racines et en particulier dans les extraits de racines d'immortelle. En outre, les inventeurs ont démontré de manière surprenante que les extraits de parties aériennes d'immortelle ne comprenaient pas d'acide léontopodique qui est donc présent uniquement dans les extraits de racines d'immortelle. Aussi, l'acide léontopodique peut être considéré comme un marqueur biologique naturel d'un extrait de racines d'immortelle.

Ainsi, avantageusement l'acide léontopodique peut être considéré comme un marqueur fiable d'un extrait de racines d'immortelle, avantageusement l'acide léontopodique est un marqueur naturel.

Dans la présente par « acide léontopodique » on entend l'acide léontopodique A habituellement trouvé dans les extraits de parties aériennes de l'edelweiss (leontopodium alpinum) de formule (I) ci-dessous :

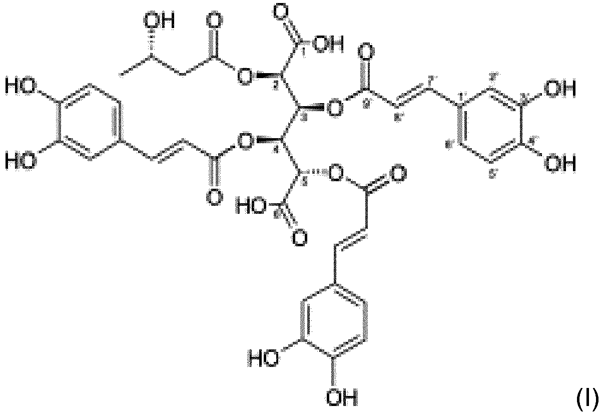

Dans la présente par « immortelle » on entend une espèce de plantes à fleurs de la famille des Asteraceae et du genre Helichrysum. En particulier, il peut s'agir de *l'Helichrysum italicum* qui peut être présente tout autour de la Méditerranée. Il peut s'agir par exemple de l'immortelle décrite dans la demande de brevet français n°01/11224.

Selon l'invention, l'extrait de racine peut être un extrait aqueux, huileux, glycolique, organique ou alcoolique de racines d'immortelle.

Dans la présente par « extrait aqueux » on entend tout extrait aqueux obtenu par tout procédé connu de l'homme du métier. Par exemple, il peut être obtenu par extraction aqueuse, par hydrodistillation, par extraction hydro-glycérinée ou hydro-alcoolique, par extraction par micro-ondes, par hyperfréquence, par extraction supercritique, par macération dans un solvant aqueux, hydroalcoolique ou hydroglycériné, par extraction assistée par ultrasons, par percolation. Le pH du solvant d'extraction peut être compris de 0 à 14. Avantageusement le pH peut être modifié afin de favoriser l'extraction de l'acide léontopodique.

Dans la présente par « extrait huileux » on entend un extrait riche en acide gras et/ou en matière grasse et/ou un extrait non hydrosoluble.

Selon l'invention, l'extrait huileux peut être tout extrait huileux obtenu par tout procédé connu de l'homme du métier. Par exemple, il peut être obtenu par extraction huileuse par micro-ondes, par hyperfréquence, par extraction supercritique, par macération dans un solvant huileux, par extraction huileuse assistée par ultra sons. Il peut s'agir par exemple d'un extrait huileux biologique d'immortelle obtenu par extraction micro-ondes assistée par ultra sons selon un procédé comprenant une première étape de broyage des racines d'immortelle suivie d'une extraction assistée par ultrasons et par micro-ondes, puis une clarification et enfin une étape de filtration afin de récupérer l'extrait huileux.

Dans la présente par «extrait alcoolique» on entend tout extrait alcoolique obtenu par tout procédé connu de l'homme du métier. Par exemple, il peut être obtenu par extraction alcoolique, par extraction hydro-alcoolique, par extraction par micro-ondes, par hyperfréquence, par extraction supercritique, par macération dans un solvant alcoolique, hydroalcoolique, par extraction assistée par ultrasons, par percolation.

Dans la présente par «extrait glycolique» on entend tout extrait glycolique obtenu par tout procédé connu de l'homme du métier. Par exemple, il peut être obtenu par extraction par propylène glycol, par dipropylène glycol, par propanediol, par glycérine, par extraction hydroglycolique, par extraction par micro-ondes, par hyperfréquence, par macération dans un solvant glycolique, hydroglycolique, par extraction assistée par ultrasons. Le pH du solvant d'extraction peut être modifié afin de favoriser l'extraction de l'acide léontopodique.

Dans la présente, par extrait « organique » on entend tout extrait organique obtenu par tout procédé connu de l'homme du métier. Par exemple, l'extrait organique peut être obtenu par extraction avec un solvant de type hexane, par exemple il peut s'agir par exemple d'un extrait obtenu par extraction par l'hexane ou cyclohexane, par extraction par dichlorométhane, par extraction par chloroforme, par extraction par micro-ondes, par hyperfréquence, par macération dans un solvant organique, par extraction assistée par ultrasons, par percolation.

Les inventeurs ont démontré de manière surprenante que l'acide léontopodique pouvait être présent à des concentrations différentes en fonction de la forme de l'extrait notamment lorsqu'il s'agit d'un extrait huileux, d'un extrait aqueux, d'un extrait glycolique, d'un extrait organique ou d'un extrait alcoolique.

Selon l'invention, l'étape de recherche de l'acide léontopodique peut être réalisée par tout procédé connu de l'homme du métier. Il peut s'agir par exemple d'une recherche par un procédé choisi dans le groupe comprenant une Chromatographie Liquide, par exemple une Chromatographie Liquide Haute Performance (HPLC), par Chromatographie Liquide Haute Performance (HPLC) couplée à une détection UV, par Chromatographie Liquide Haute Performance (HPLC) couplée à un spectromètre de masse, par Chromatographie Liquide Haute Performance (HPLC) couplée à un détecteur DEDL, Chromatographie Liquide Haute Performance (HPLC) couplée à un dispositif de Résonance Magnétique Nucléaire, Chromatographie Liquide Haute Performance (HPLC) couplée à un spectromètre Infra-rouge, d'un procédé comprenant une Chromatographie à Ultra Haute Performance (UHPLC), d'un procédé chromatographique, par exemple par Chromatographie sur Couche Mince Haute Performance (HPTLC), par Chromatographie sur Couche Mince Haute Performance (HPTLC) couplée à un spectromètre de masse, par Chromatographie sur Couche Mince, par Chromatographie par Fluide Supercritique (SFC), par Chromatographie par Fluide Supercritique (SFC) couplée à un spectromètre de masse, par Mobilité Ionique, par Mobilité Ionique couplée à un spectromètre de masse, par Electrophorèse capillaire, d'un procédé choisi dans le groupe comprenant une Chromatographie en Phase Gazeuse, par exemple, une Chromatographie en Phase Gazeuse (CPG), une Chromatographie en Phase Gazeuse couplée à un détecteur à ionisation de flamme, une Chromatographie en Phase Gazeuse couplée à un spectromètre de masse, d'un procédé d'identification directe, par exemple par Résonance Magnétique Nucléaire, par Spectrométrie Infrarouge, par Spectrométrie de masse à introduction directe.

Il peut s'agir par exemple d'une Chromatographie Liquide Haute Performance (HPLC) utilisant un gradient d'élution à base d'eau, par exemple de l'eau avec un pH compris de 1,5 à 3, de préférence de 2,1, et d'alcool, par exemple avec du méthanol, ou un gradient d'acétonitrile. Par exemple il peut s'agir d'une Chromatographie Liquide Haute Performance (HPLC) utilisant par exemple une colonne d'élution disponible dans le commerce, par exemple une colonne d'élution commercialisée par la société Phenomenex, par la société Shimadzu et/ou toute colonne d'élution connu de l'homme du métier adaptée.

Il peut s'agir par exemple d'une Chromatographie Liquide Haute Performance (HPLC) avec un gradient d'élution tel que décrit dans les tableaux 1 et 2 ci-dessous.

**Tableau 1 : gradient d'élution eau /acétonitrile**

| Temps | Eau acidifiée acide formique à 0,1 % (pH proche 2,1) | Acétonitrile (ACN) |
|---|---|---|
| 0 | 70% | 30% |
| 20 mn | 50% | 50% |
| 25 mn | 50% | 50% |
| 30 mn | 20% | 80% |
| 45 mn | 20% | 80% |
| 47 mn | 70% | 30% |
| 60 mn | 70% | 30% |

**Tableau 2 : gradient d'élution eau /méthanol**

| Temps | Eau acidifiée acide formique à 0,1 % (pH proche 2,1) | Méthanol (MeOH) |
|---|---|---|
| 0 | 70% | 30% |
| 5,95 mn | 50% | 50% |
| 7,44 mn | 50% | 50% |
| 8,93 mn | 20% | 80% |
| 13,40 mn | 20% | 80% |
| 13,99 mn | 70% | 30% |
| 35 mn | 70% | 30% |

Bien entendu l'homme du métier, de part ces connaissances générales saura adapter le gradient d'élution et/ou le choix des solvants en fonction de la forme et/ou le type d'extrait obtenu afin d'identifier l'acide léontopodique.

La présente invention a également pour objet l'utilisation de l'acide léontopodique comme marqueur de la présence d'un extrait de racines d'immortelle dans un échantillon.

Dans cette utilisation, l'extrait est tel que défini ci-dessus. Il peut s'agir par exemple d'un extrait choisi parmi un extrait huileux, un extrait aqueux, un extrait alcoolique, un extrait organique, un extrait glycolique de racines d'immortelle ou un mélange de ceux-ci.

Selon l'invention, l'échantillon peut être tout échantillon connu de l'homme du métier. Il peut s'agir par exemple d'un échantillon issu d'un produit cosmétique et/ou un échantillon issu d'un produit pharmaceutique, d'un échantillon résultant d'un mélange d'extraits de plantes.

Il peut s'agir par exemple d'un échantillon issu d'un produit cosmétique, par exemple tout produit cosmétique, quelque soit sa forme, connu de l'homme du métier. Il peut s'agir par exemple d'un échantillon issu d'une composition cosmétique pour application topique, par exemple sur la peau, sur les muqueuses ; pour application capillaire, par exemple sur les cheveux, les cils, les poils. La composition cosmétique ou dermatologique peut être, par exemple une composition pour le soin du visage, du corps, des cheveux, par exemple des compositions pour le visage et/ou le corps et/ou les cheveux.

Selon l'invention, lorsque l'échantillon est issu d'un produit cosmétique il peut comprendre préalablement à l'étape de recherche de l'acide léontopodique, une étape de préparation de l'échantillon. Il peut s'agir par exemple d'une étape de dilution de la composition dans une solution alcoolique afin d'obtenir une solution / extrait alcoolique de ladite composition cosmétique.

Il peut s'agir par exemple d'une dilution dans tout solvant organique adapté connu de l'homme du métier, par exemple de l'hexane.

Il peut s'agir par exemple d'une dilution dans une solution aqueuse, il peut s'agir par exemple d'une extraction liquide/liquide ou d'une extraction solide/liquide, il peut s'agir par exemple d'une purification par extraction sur phase solide (SPE).

Par exemple, une solution/extrait d'une composition cosmétique peut être obtenu par exemple selon l'un des protocoles suivants. Par exemple pour une composition cosmétique aqueuse, par exemple un tonique, la composition cosmétique peut être mise avec un solvant de dilution, par exemple un mélange eau/éthanol 50:50 en volume dans une fiole jaugée, par exemple de 20mL, puis agiter, par exemple par agitation magnétique, par exemple pendant 20min, le mélange peut être ensuite filtré, par exemple sur filtre nylon, par exemple un filtre nylon disponible dans le commerce, par commercialisé par la société Millipore d'un diamètre de pore de 0,45µm. La quantité de composition cosmétique utilisée peut être par exemple de 10g, auquel peut être ajouté le solvant de dilution jusqu'à obtenir le volume désiré, par exemple 20 ml.

Par exemple, pour une composition cosmétique type émulsion, moussant, la composition cosmétique peut être mise avec un solvant de dilution, par exemple un mélange eau/éthanol 50:50 en volume dans une fiole jaugée, par exemple de 20mL, puis agiter, par exemple par agitation magnétique, par exemple pendant 20min, par exemple le mélange peut être ensuite filtré, par exemple sur filtre nylon, par exemple un filtre nylon disponible dans le commerce, par commercialisé par la société Millipore d'un diamètre de pore de 0.45µm. La quantité de composition cosmétique utilisée peut être par exemple de 5g, auquel peut être ajouté le solvant de dilution jusqu'à obtenir le volume désiré, par exemple 20 ml.

Bien entendu l'homme du métier, de part ces connaissances générales saura adapter la préparation de l'échantillon en fonction du type de composition cosmétique et/ou du procédé de recherche de l'acide léontopodique.

Les inventeurs ont démontré de manière surprenante et contrairement à l'enseignement de l'art que l'acide léontopodique est un marqueur biologique naturel d'un extrait de racines, en particulier d'un extrait de racine d'immortelle.

Les inventeurs ont également démontré et mis en oeuvre un procédé d'identification dans un échantillon d'un extrait de racines via la recherche de l'acide léontopodique.

Les inventeurs ont également démontré de manière surprenante que l'acide léontopodique permet avantageusement d'identifier la présence d'un extrait de racines, notamment d'immortelle dans un échantillon.

La présente description a donc également pour objet l'utilisation de l'acide léontopodique pour l'identification dans un échantillon de la présence d'un extrait de racines.

D'autres avantages pourront également apparaître à l'homme du métier à la lecture des exemples ci-dessous donnés à titre illustratif.

### Brève description des figures

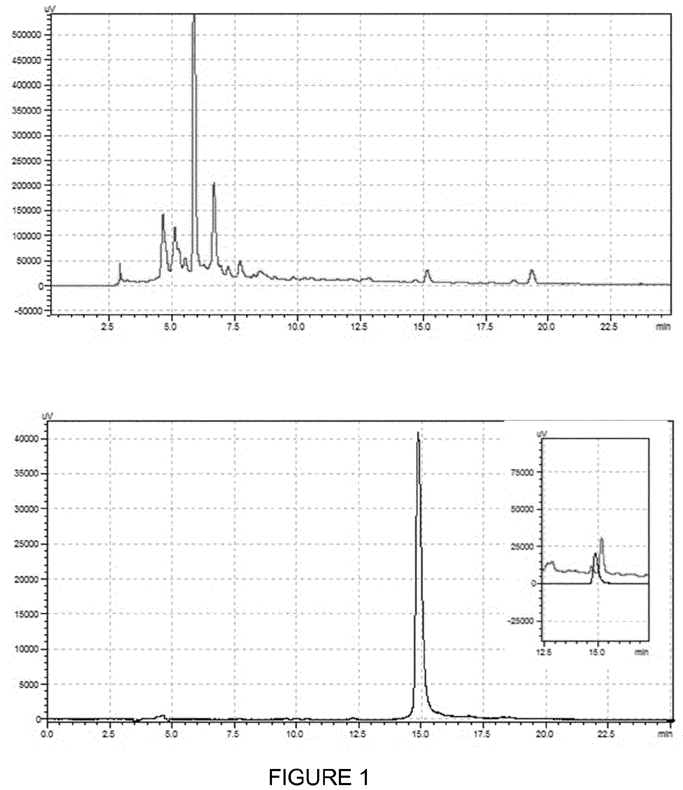

La figure 1 représente deux chromatogrammes d'élution. En haut, il s'agit du chromatogramme d'élution en fonction du temps en minutes d'un extrait aqueux de parties aériennes d'immortelle. Le chromatogramme du bas représente le chromatogramme d'élution en fonction du temps en minutes d'une solution aqueuse comprenant de l'acide léontopodique.

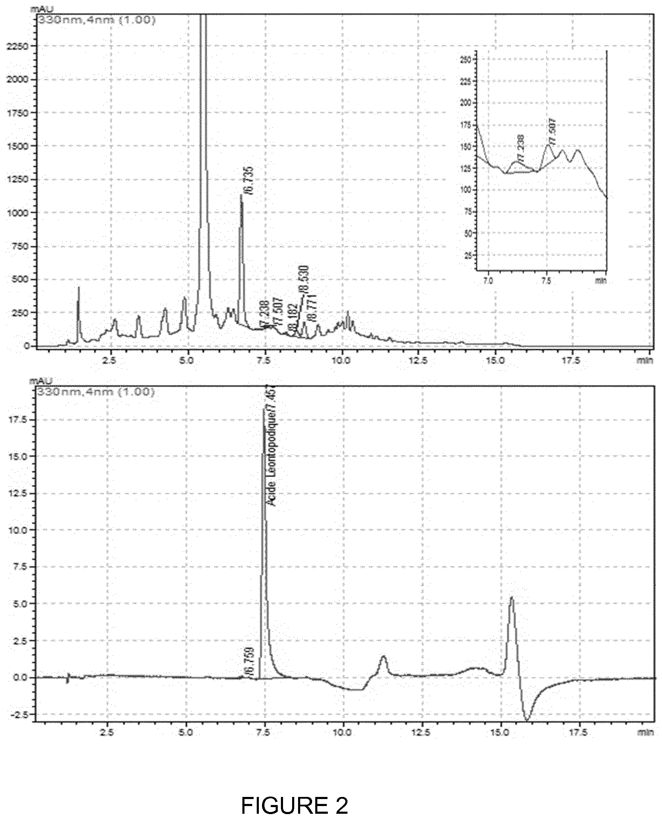

La figure 2 représente deux chromatogrammes d'élution. En haut, il s'agit du chromatogramme d'élution en fonction du temps en minutes d'un extrait hydroalcoolique de parties aériennes d'immortelle. Le chromatogramme du bas représente le chromatogramme d'élution en fonction du temps en minutes d'une solution aqueuse comprenant de l'acide léontopodique.

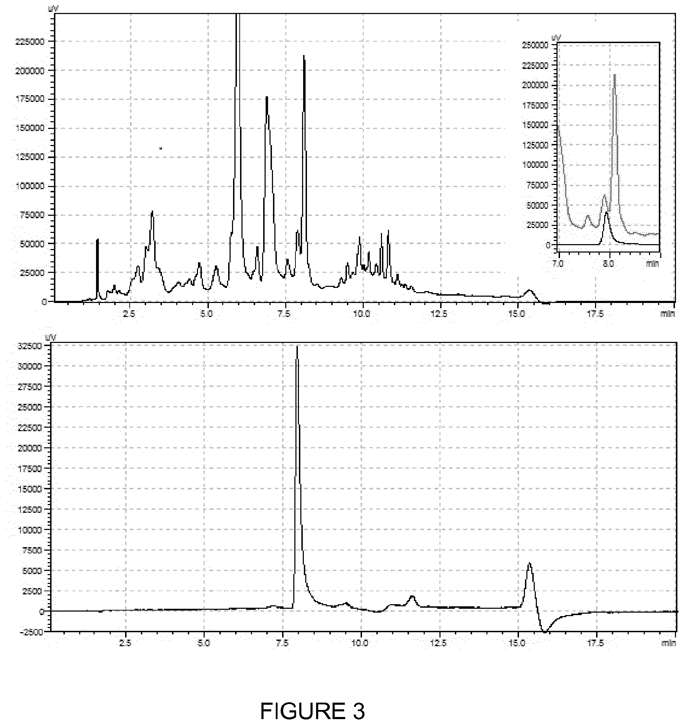

La figure 3 représente deux chromatogrammes d'élution. En haut, il s'agit du chromatogramme d'élution en fonction du temps en minutes d'un extrait hydroalcoolique de racines d'immortelle. Le chromatogramme du bas représente le chromatogramme d'élution en fonction du temps en minutes d'une solution aqueuse comprenant de l'acide léontopodique.

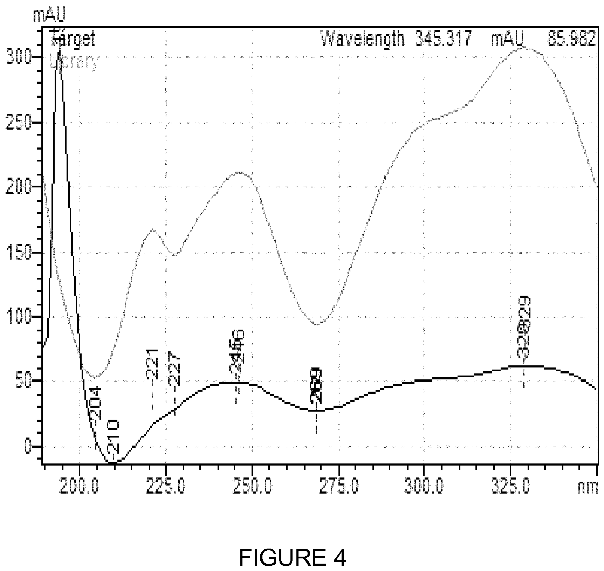

La figure 4 représente un diagramme correspondant au spectre UV à une longueur de 330nm d'un extrait hydroalcoolique de racines d'immortelle (courbe du bas) et d'une solution aqueuse comprenant de l'acide léontopodique.

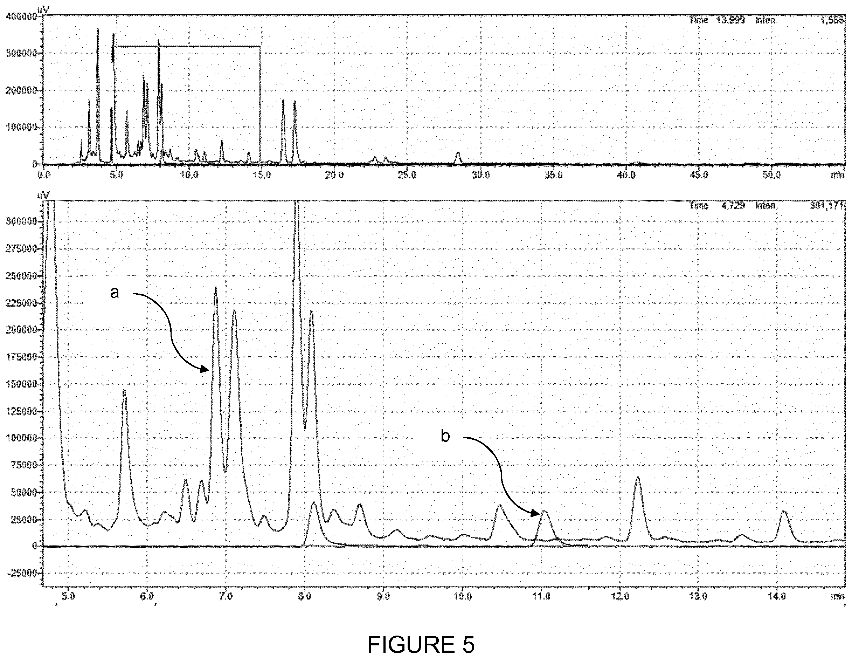

La figure 5 représente deux chromatogrammes d'élution. En haut, il s'agit du chromatogramme d'élution en fonction du temps en minutes d'un extrait hydroalcoolique de rhizomes d'iris. Le chromatogramme du bas représente le chromatogramme d'élution en fonction du temps en minutes d'une solution aqueuse comprenant de l'acide léontopodique (b) superposé avec le chromatogramme d'élution de l'extrait de rhizomes d'iris (a).

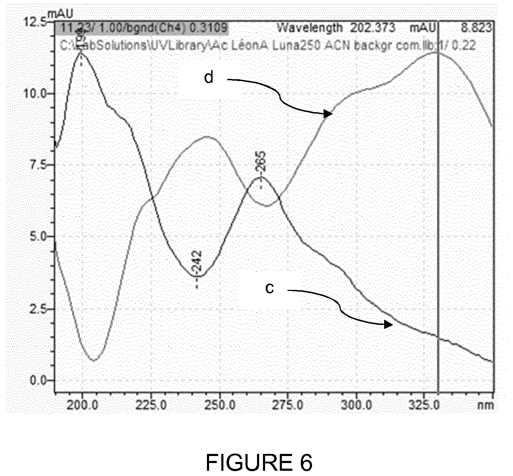

La figure 6 représente un spectre UV enregistré entre les longueurs d'onde 190nm et 350 nm d'un extrait hydroalcoolique de rhizomes d'iris (courbe du bas) (c) et d'une solution aqueuse comprenant de l'acide léontopodique (d).

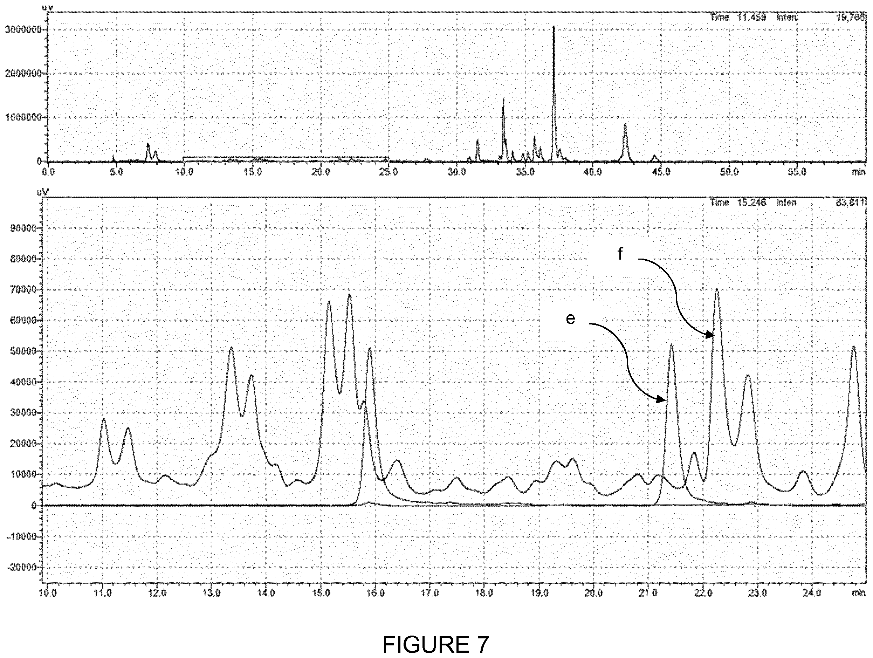

La figure 7 représente deux chromatogrammes d'élution. En haut, il s'agit du chromatogramme d'élution en fonction du temps en minutes d'un extrait hydroalcoolique de racines d'angélique. Le chromatogramme du bas représente le chromatogramme d'élution en fonction du temps en minutes d'une solution aqueuse comprenant de l'acide léontopodique (e) superposé avec le chromatogramme d'élution de l'extrait de racines d'angélique (f).

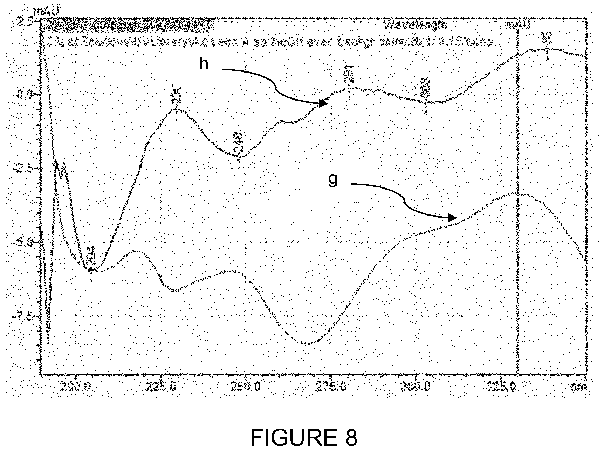

La figure 8 représente un spectre UV enregistré entre les longueurs d'ondes 190nm et d'un extrait hydroalcoolique de racines d'angélique (courbe du bas, (h)) et d'une solution aqueuse comprenant de l'acide léontopodique (courbe supérieure, (g)).

### EXEMPLES

### Exemple 1 : identification de marqueur d'extrait de racines

Dans le présent exemple, une analyse par Chromatographie Liquide Haute performance couplée à un spectromètre UV de différents extraits d'immortelles a été réalisée. Pour ce faire les réactifs suivants ont été utilisés :
- Acide formique ACS reagent commercialisé par Sigma-Aldrich, référence 251364
- Méthanol grade HPLC commercialisé par Sigma-Aldrich, référence 34860
- Acétonitrile grade HPLC commercialisé par Sigma-Aldrich, référence 34851
- Ethanol pureté 96%
- Eau distillée.

Les extraits ont été obtenus grâce à une cuve à ultrasons commercialisée par la société Reus. La chaine de chromatographie liquide utilisée pour les essais était un modèle UFLC-XR commercialisée par la marque Shimadzu équipée d'un détecteur DAD. Les différents extraits ont été préalablement filtrés sur filtres en nylon 0,45µm, commercialisés par la société Millipore avant injection HPLC. La quantification a été réalisée via un spectre UV calibré à une longueur d'onde de 330 nm. Il a été noté que l'acide léontopodique possède un spectre UV avec un maximum d'absorption à 330nm.

Les extraits étudiés et analysés sont résumés dans le tableau 3 ci-dessous :

**Tableau 3 : composition des extraits et résultats associés**

| **Extraits** | **Plante** | **Parties utilisées** | **Technique d'extraction** | **Acide léontopodique A** |
|---|---|---|---|---|
| **Extrait 1** | *Helichrysum italicum* | Parties aériennes | Solvant : eau | Non Détecté |
| | | | Procédé: fournisseur, 10 à 12% de végétal | |
| **Extrait 2** | *Helichrysum italicum* | Racines | Solvant : eau | 1 à 3 ppm |
| | | | Végétal broyé 10mm. 16% de végétal. | |
| | | | US 30 min, enceinte thermostatée à 50°C | |
| **Extrait 3** | *Helichrysum italicum* | Racines | Solvant : eau/éthanol (50/50, v/v) | 5 ppm |
| | | | Végétal broyé 10mm. 10% de végétal. | |
| | | | US 30 min, enceinte thermostatée à 10°C | |
| **Extrait 4** | *Helichrysum italicum* | Racines | Solvant : eau/éthanol (50/50, v/v) | 20 ppm |
| | | | Végétal broyé 10mm. 10% de végétal. | |
| | | | US 30 min, enceinte thermostatée à 50°C | |
| **Extrait 5** | *Helichrysum italicum* | Parties aériennes | Solvant : eau/éthanol (50/50, v/v) | Non Détecté |
| | | | Végétal broyé 2mm. 10% de végétal. | |
| | | | US 30 min, enceinte thermostatée à 50°C | |

Les procédés d'obtentions des extraits mentionnés dans le tableau ci-dessus sont détaillés ci-dessous. En particulier, les extraits hydroalcooliques de parties aériennes ont été obtenus selon le procédé suivant : broyage du végétal sec à 2 mm, mise du végétal sec broyé (10%) et le support (eau/éthanol 45:45) dans une cuve à ultrasons dont la double enveloppe a été chauffée à 50°C, fermeture hermétique de la cuve à ultrasons et application des ultrasons pendant 30 minutes sous agitation, séparation solide / liquide, filtration sur coton 100%, filtration sur un papier filtre en cellulose commercialisé par la société Sodipro, avec des pores de diamètres compris de 12 à 15 µm, puis filtration sur un filtre Sartolab en polyethersulfone commercialisé par la société Sartorius, avec des pores d'un diamètre de 0,22 µm. La quantité de végétal utilisée était de 50g, la quantité d'eau : 225g et la quantité d'alcool, à savoir de l'éthanol, de 225g.

En outre, les extraits hydroalcooliques de racines ont été obtenus selon les procédés suivants :
- broyage du végétal sec à 10 mm, mise du végétal sec broyé (10%) et le support (eau/éthanol 45:45) dans une cuve à ultrasons dont la double enveloppe a été chauffée à 10°C, fermeture hermétique de la cuve à ultrasons et application des ultrasons pendant 30 minutes sous agitation, séparation solide / liquide, filtration sur coton 100%, filtration sur un papier filtre en cellulose commercialisé par la société Sodipro, avec des pores de diamètres compris de 12 à 15 µm, puis filtration sur un filtre Sartolab en polyethersulfone commercialisé par la société Sartorius avec des pores d'un diamètre de 0,22 µm. La quantité de végétal utilisée était de 50g, la quantité d'eau : 225g et la quantité d'alcool, à savoir de l'éthanol de 225g, ou
- broyage du végétal sec à 10 mm, mise du végétal sec broyé (10%) et le support (eau/éthanol 45:45) dans une cuve à ultrasons dont la double enveloppe a été chauffée à 50°C, fermeture hermétique de la cuve à ultrasons et application des ultrasons pendant 30 minutes sous agitation, séparation solide / liquide, filtration sur coton 100%, filtration sur un papier filtre en cellulose commercialisé par la société Sodipro, avec des pores de diamètres compris de 12 à 15 µm, puis filtration sur un filtre Sartolab en polyethersulfone commercialisé par la société Sartorius avec des pores d'un diamètre de 0,22 µm. La quantité de végétal utilisée était de 50g, la quantité d'eau : 225g et la quantité d'alcool, à savoir de l'éthanol de 225g.

Les extraits aqueux de racines d'immortelle ont été obtenus selon le procédé suivant : broyage du végétal sec à 10 mm, mise du végétal sec broyé (16%) et de l'eau (84%) dans une cuve à ultrasons dont la double enveloppe a été chauffée à 50°C, fermeture hermétique de la cuve à ultrasons et application des ultrasons pendant 30 minutes sous agitation, séparation solide / liquide, filtration sur coton 100%, filtration sur un papier filtre en cellulose commercialisé par la société Sodipro, avec des pores de diamètres compris de 12 à 15 µm, puis filtration sur un filtre Sartolab en polyethersulfone commercialisé par la société Sartorius, avec des pores d'un diamètre de 0,22 µm. Puis des conservateurs : 0,3% de benzoate de sodium et 0,1% d'acide citrique ont été ajoutés. L'ensemble a été homogénéisé par agitation magnétique et il a été vérifié que le pH était entre 3,8 et 4,5 La quantité de végétal utilisée était de 80g et la quantité d'eau 420g.

Les extraits aqueux de parties aériennes d'immortelle ont été obtenus selon le procédé suivant : broyage du végétal sec à 10 mm, mise du végétal sec broyé (10%) et de l'eau (90%) dans une cuve à ultrasons dont la double enveloppe a été chauffée à 50°C, fermeture hermétique de la cuve à ultrasons et application des ultrasons pendant 30 minutes sous agitation, séparation solide / liquide, filtration sur coton 100%, filtration sur un papier filtre en cellulose commercialisé par la société Sodipro, avec des pores de diamètres compris de 12 à 15 µm, puis filtration sur un filtre Sartolab en polyethersulfone commercialisé par la société Sartorius, avec des pores d'un diamètre de 0,22 µm. Puis des conservateurs : 0,3% de benzoate de sodium et 0,1% d'acide citrique ont été ajoutés. L'ensemble a été homogénéisé par agitation magnétique et il a été vérifié que le pH était entre 3,8 et 4,5. La quantité de végétal utilisée était de 50g et la quantité d'eau de 450g.

L'analyse de l'extrait 1 a été réalisée suivant le gradient décrit dans le tableau 4 (différent des 4 autres extraits pour éviter une co-élution) à l'aide d'une colonne Luna C18 (250mm x 4,6mm x 5µm) commercialisée par la société Phenomenex. Le volume d'injection était de 20µL et le débit d'élution de 0,7ml/min. Le tableau 4 ci-dessous décrit le gradient d'élution.

**Tableau 4 : gradient d'élution d'analyse de l'extrait 1**

| Temps | Eau acidifiée acide formique à 0,1% (pH proche 2,1) | Acétonitrile (ACN) |
|---|---|---|
| 0 | 70% | 30% |
| 20 mn | 50% | 50% |
| 25 mn | 50% | 50% |
| 30 mn | 20% | 80% |
| 45 mn | 20% | 80% |
| 47 mn | 70% | 30% |
| 60 mn | 70% | 30% |

Pour les extraits 2, 3, 4 et 5, ils ont été analysés suivant le gradient d'élution mentionné dans le tableau 5 à l'aide d'une colonne XRODS II (100mm x 3mm x 2,2µm) commercialisée par la société Shimadzu. Le volume d'injection était de 5µL et le débit d'élution de 0,4ml/min.

**Tableau 5 : gradient d'élution d'analyse des extraits 2 à 5**

| Temps | Eau acidifiée acide formique à 0,1 % (pH proche 2,1) | Méthanol (MeOH) |
|---|---|---|
| 0 | 70% | 30% |
| 5,95 mn | 50% | 50% |
| 7,44 mn | 50% | 50% |
| 8,93 mn | 20% | 80% |
| 13,40 mn | 20% | 80% |
| 13,99 mn | 70% | 30% |
| 35 mn | 70% | 30% |

Lors de chaque analyse, un standard d'acide léontopodique à 20 ppm a été injecté afin de confirmer le temps de rétention et le spectre UV à 330 nm.

La figure 1 représente en haut un chromatogramme obtenu à partir de l'extrait 1 correspondant à un extrait aqueux de parties aériennes d'immortelle et le chromatogramme du bas un chromatogramme obtenu à partir d'une solution aqueuse comprenant de l'acide léontopodique. Tel que démontré sur cette figure, l'extrait aqueux de parties aérienne d'immortelle ne présente sur le chromatogramme aucun pic susceptible de correspondre au pic d'élution de l'acide léontopodique. En d'autres termes, l'extrait aqueux de parties aériennes d'immortelle ne comprend pas d'acide léontopodique.

La figure 2 représente en haut un chromatogramme obtenu à partir de l'extrait 5 correspondant à un extrait hydroalcoolique de parties aériennes d'immortelle et le chromatogramme du bas un chromatogramme obtenu à partir d'une solution aqueuse comprenant de l'acide léontopodique. Tel que démontré sur cette figure, l'extrait hydroalcoolique de parties aérienne d'immortelle ne présente sur le chromatogramme aucun pic susceptible de correspondre au pic d'élution de l'acide léontopodique. En d'autres termes, l'extrait aqueux de parties aériennes d'immortelle ne comprend pas d'acide léontopodique.

La figure 3 représente en haut un chromatogramme obtenu à partir de l'extrait 4 correspondant à un extrait eau / éthanol (50/50) de racines d'immortelle et le chromatogramme du bas un chromatogramme obtenu à partir d'une solution aqueuse comprenant de l'acide léontopodique. Tel que démontré sur cette figure, l'extrait de racines d'immortelle comprend un pic d'élution identique à celui de la solution comprenant de l'acide léontopodique seul situé à 7,95 minutes. En particulier, tel que présenté sur cette figure, un agrandissement du spectre entre 7 et 9 minutes et superposition des chromatogrammes démontrent clairement que l'extrait de racines comprend un pic d'élution correspondant au pic d'élution de l'acide léontopodique.

Cet exemple démontre donc clairement que des extraits de racines d'immortelle comprennent de l'acide léontopodique alors que des extraits de parties aériennes, quelque soit la forme de l'extrait, d'immortelle ne comprennent pas d'acide léontopodique. Cet exemple démontre donc clairement que l'acide léontopodique est un marqueur d'un extrait de racines d'immortelle.

En outre, la figure 4 représente un spectre UV comprenant en bas un spectre obtenu à partir de l'extrait 4 correspondant à un extrait eau / éthanol (50/50) de racines d'immortelle et le spectre du bas obtenu à partir d'une solution aqueuse comprenant de l'acide léontopodique. Tel que démontré sur cette figure, l'extrait de racines d'immortelle comprend un spectre similaire à celui de la solution comprenant de l'acide léontopodique seul. En particulier, les maximums et minimums locaux d'absorption des deux spectres correspondent, la différence d'intensité étant due à un effet de matrice avec l'extrait 4.

Cet exemple démontre donc clairement que des extraits de racines d'immortelle comprennent de l'acide léontopodique alors que des extraits de parties aériennes d'immortelle, quelque soit la forme de l'extrait, ne comprennent pas d'acide léontopodique. Cet exemple démontre donc clairement que l'acide léontopodique est un marqueur d'un extrait de racines d'immortelle.

### Exemple 2 : comparaison de différents extraits de racines/rhizomes

Dans le présent exemple, une analyse par Chromatographie Liquide Haute performance couplée à un spectromètre UV de différents extraits a été réalisée comme décrit dans l'exemple 1 ci-dessus.

Les extraits étudiés et analysés sont résumés dans le tableau 6 ci-dessous :

**Tableau 6 : composition des extraits et résultats associés**

| **Extrait** | **Plante** | **Parties utilisées** | **Technique d'extraction** | **Acide léontopodique** |
|---|---|---|---|---|
| **Extrait 6** | *Iris florentina* | Rhizomes | Solvant : eau/éthanol (50/50, v/v) | Non Détecté |
| | | | Végétal broyé 10mm. 10% de végétal. | |
| | | | US 30 min, enceinte thermostatée à 50°C | |
| **Extrait 7** | *Angelica archangelica* | Racines | Solvant : eau/éthanol (50/50, v/v) | Non Détecté |
| | | | Végétal broyé 10mm. 10% de végétal. | |
| | | | US 30 min, enceinte thermostatée à 50°C | |

Les procédés d'obtentions des extraits mentionnés dans le tableau ci-dessus étaient identiques à ceux de l'exemple 1.

L'analyse de l'extrait 6 a été réalisée suivant le gradient décrit dans le tableau 7 à l'aide d'une colonne Luna C18 (250mm x 4,6mm x 5µm) commercialisée par la société Phenomenex. Le volume d'injection était de 20µL et le débit d'élution de 0,7ml/min. Le tableau 7 ci-dessous décrit le gradient d'élution.

**Tableau 7 : gradient d'élution d'analyse de l'extrait 6**

| Temps | Eau acidifiée acide formique à 0,1% (pH proche 2,1) | Acétonitrile (ACN) |
|---|---|---|
| 0 | 70% | 30% |
| 20 mn | 50% | 50% |
| 25 mn | 50% | 50% |
| 30 mn | 20% | 80% |
| 45 mn | 20% | 80% |
| 47 mn | 70% | 30% |
| 60 mn | 70% | 30% |

L'analyse de l'extrait 7 a été réalisée suivant le gradient décrit dans le tableau 8 à l'aide d'une colonne Luna C18 (250mm x 4,6mm x 5µm) commercialisée par la société Phenomenex. Le volume d'injection était de 20µL et le débit d'élution de 0,7ml/min. Le tableau 8 ci-dessous décrit le gradient d'élution.

**Tableau 8 : gradient d'élution d'analyse de l'extrait 7**

| Temps | Eau acidifiée acide formique à 0,1% (pH proche 2,1) | MeOH |
|---|---|---|
| 0 | 70% | 30% |
| 20 mn | 50% | 50% |
| 25 mn | 50% | 50% |
| 30 mn | 20% | 80% |
| 45 mn | 20% | 80% |
| 47 mn | 70% | 30% |
| 60 mn | 70% | 30% |

Lors de chaque analyse, un standard d'acide léontopodique à 20 ppm a été injecté afin de confirmer le temps de rétention et le spectre UV à 330 nm.

La figure 5 représente en haut un chromatogramme obtenu à partir de l'extrait 6 correspondant à un extrait hydroalcoolique de rhizomes d'iris et le chromatogramme du bas un chromatogramme obtenu à partir d'une solution aqueuse comprenant de l'acide léontopodique (b) superposé avec le chromatogramme obtenu à partir de l'extrait 6 (a). Tel que démontré sur cette figure, l'extrait de rhizomes d'iris ne présente sur le chromatogramme aucun pic susceptible de correspondre au pic d'élution de l'acide léontopodique à 11,05 minutes de temps d'élution. En d'autres termes, l'extrait de rhizomes d'iris ne comprend pas d'acide léontopodique.

La figure 6 représente un spectre UV obtenu à un temps d'élution de 11,05 min comprenant deux spectres obtenus respectivement à partir de l'extrait hydroalcoolique de rhizomes d'iris (c) et d'une solution aqueuse comprenant de l'acide léontopodique (d). Tel que démontré sur cette figure, l'extrait de rhizomes d'iris ne possède pas un spectre similaire à celui de la solution comprenant l'acide léontopodique. En particulier, les minimums et maximums locaux d'absorption des deux spectres ne correspondent pas.

La figure 7 représente en haut un chromatogramme obtenu à partir de l'extrait 7 correspondant à un extrait hydroalcoolique de racines d'angélique et le chromatogramme du bas un chromatogramme obtenu à partir d'une solution aqueuse comprenant de l'acide léontopodique (e) superposé avec le chromatogramme obtenu à partir de l'extrait 7 (f). Tel que démontré sur cette figure, l'extrait hydroalcoolique de racines d'angélique ne présente sur le chromatogramme aucun pic susceptible de correspondre au pic d'élution de l'acide léontopodique à 21,45 min de temps d'élution. En d'autres termes, l'extrait de racines d'angélique ne comprend pas d'acide léontopodique.

La figure 8 représente un spectre UV obtenu à un temps d'élution de 21,45min comprenant deux spectres obtenus respectivement à partir de l'extrait hydroalcoolique de racines d'angélique (h) et d'une solution aqueuse comprenant de l'acide léontopodique (g). Tel que démontré sur cette figure, l'extrait de racines d'angélique ne possède pas un spectre similaire à celui de la solution comprenant l'acide léontopodique. En particulier, les minimums et maximums locaux d'absorption des deux spectres ne correspondent pas.

Cet exemple démontre donc clairement que des extraits de racines, autre que d'immortelle, par exemple des extraits de racines d'angélique, des extraits de rhizomes d'iris ne comprennent pas d'acide léontopodique.

Cet exemple démontre donc clairement que l'acide léontopodique est un marqueur d'un extrait de racines d'immortelle.

## Revendications

1. Procédé d'identification de l'origine d'un extrait de racine, ledit procédé comprenant une étape de recherche dans ledit extrait de racines d'acide léontopodique, la présence d'acide léontopodique dans ledit extrait indiquant qu'il s'agit d'un extrait de racine d'immortelle.

2. Procédé d'identification selon la revendication 1, dans lequel l'extrait de racines est un extrait de racines *d'Helichrysum italicum.*

3. Procédé selon la revendication 1 ou 2, dans lequel l'extrait est choisi parmi un extrait aqueux, huileux, glycolique, organique ou alcoolique de racines d'immortelle.

4. Procédé selon l'une quelconque des revendications précédente dans lequel la recherche de l'acide léontopodique est effectuée par Chromatographie Liquide Haute Performance (HPLC), par Chromatographie Liquide Haute Performance (HPLC) couplée à une détection UV, par Chromatographie Liquide Haute Performance (HPLC) couplée à un spectromètre de masse, par Chromatographie Liquide Haute Performance (HPLC) couplée à un détecteur DEDL, Chromatographie Liquide Haute Performance (HPLC) couplée à un dispositif de Résonance Magnétique Nucléaire, par Chromatographie Liquide Haute Performance (HPLC) couplée à un spectromètre Infra-rouge, par Chromatographie à Ultra Haute Performance (UHPLC), par Chromatographie sur Couche Mince Haute Performance (HPTLC), par Chromatographie sur Couche Mince Haute Performance (HPTLC) couplée à un spectromètre de masse, par Chromatographie sur Couche Mince, par Chromatographie par Fluide Supercritique (SFC), par Chromatographie par Fluide Supercritique (SFC) couplée à un spectromètre de masse, par Mobilité Ionique, par Mobilité Ionique couplée à un spectromètre de masse, par Electrophorèse capillaire, par Chromatographie en Phase Gazeuse (CPG), par Chromatographie en Phase Gazeuse couplée à un détecteur à ionisation de flamme, par Chromatographie en Phase Gazeuse couplée à un spectromètre de masse, par Résonance Magnétique Nucléaire, par Spectrométrie Infrarouge, par Spectrométrie de masse à introduction directe.

5. Utilisation de l'acide léontopodique comme marqueur de la présence d'un extrait de racines d'immortelle dans un échantillon.

6. Utilisation selon la revendication 5, dans laquelle l'extrait est un extrait huileux de racines d'immortelle.

7. Utilisation selon la revendication 5, dans laquelle l'extrait est un extrait aqueux de racines d'immortelle.

8. Utilisation selon la revendication 5, dans laquelle l'extrait est un extrait alcoolique de racines d'immortelle.

9. Utilisation selon la revendication 5, dans laquelle l'extrait est un extrait glycolique de racines d'immortelle.

10. Utilisation selon la revendication 5, dans laquelle l'extrait est un extrait organique de racines d'immortelle.

11. Utilisation selon la revendication 5, dans laquelle l'échantillon est un prélèvement d'un produit cosmétique.

## Patentansprüche

1. Verfahren zur Identifizierung des Ursprungs eines Wurzelextrakts, wobei das Verfahren einen Schritt des Suchens in dem Extrakt von Leontopodiumsäurewurzeln umfasst, wobei die Anwesenheit von Lentopodiumsäure in dem Extrakt anzeigt, dass es sich um einen Strohblumenwurzelextrakt handelt.

2. Verfahren zur Identifizierung nach Anspruch 1, wobei der Wurzelextrakt ein Extrakt von *Helichrysum italicum-Wurzeln* ist.

3. Verfahren nach Anspruch 1 oder 2, wobei der Extrakt ausgewählt ist aus einem wässrigen, öligen, glykolischen, organischen oder alkoholischen Strohblumenwurzelextrakt.

4. Verfahren nach einem der beliebigen der vorhergehenden Ansprüche, wobei die Suche nach Leontopodiumsäure durchgeführt wird durch Hochleistungsflüssigkeitschromatographie (HPLC), durch Hochleistungsflüssigkeitschromatographie (HPLC), gekoppelt mit UV-Nachweis, durch Hochleistungsflüssigkeitschromatographie (HPLC), gekoppelt mit einem Massenspektrometer, durch Hochleistungsflüssigkeitschromatographie (HPLC), gekoppelt mit einem DEDL-Detektor, Hochleistungsflüssigkeitschromatographie (HPLC), gekoppelt mit einer Kernspintomographie-Vorrichtung, durch Hochleistungsflüssigkeitschromatographie (HPLC), gekoppelt mit einem Infrarotspektrometer, durch Ultrahochleistungschromatogralphie (UHPLC), durch Hochleistungs-Dünnschichtchromatograhie (HPTLC), durch Hochleistungs-Dünnschichtchromatograhie (HPTLC), gekoppelt mit einem Massenspektrometer, durch Dünnschichtchromatograhie, durch Überkritische Fluidchromatographie (SFC), durch Überkritische Fluidchromatographie (SFC), gekoppelt mit einem Massenspektrometer, durch Ionenmobilität, durch Ionenmobilität, gekoppelt mit einem Massenspektrometer, durch Kapillarelektrophorese, durch Gasphasenchromatographie (CPG), durch Gasphasenchromatographie (CPG), gekoppelt mit einem Flammenionisationsdetektor, durch Gasphasenchromatographie, gekoppelt mit einem Massenspektrometer, durch Kernspintomographie, durch Infrarotspektrometrie, durch Massenspektrometrie mit direkter Einführung.

5. Verwendung der Leontopodiumsäure als Marker der Anwesenheit eines Strohblumenwurzelextrakts in einer Probe.

6. Verwendung nach Anspruch 5, wobei der Extrakt ein öliger Extrakt von Strohblumenwurzeln ist.

7. Verwendung nach Anspruch 5, wobei der Extrakt ein wässriger Extrakt von Strohblumenwurzeln ist.

8. Verwendung nach Anspruch 5, wobei der Extrakt ein alkoholischer Extrakt von Strohblumenwurzeln ist.

9. Verwendung nach Anspruch 5, wobei der Extrakt ein glykolischer Extrakt von Strohblumenwurzeln ist.

10. Verwendung nach Anspruch 5, wobei der Extrakt ein organischer Extrakt von Strohblumenwurzeln ist.

11. Verwendung nach Anspruch 5, wobei die Probe eine Entnahme eines kosmetischen Produkts ist.

## Claims

1. A method of identifying the origin of a root extract, said method comprising a step of searching leontopodic acid in said extract roots, the presence of leontopodic acid in said extract indicating that it is a an root extract of everlasting.

2. An identification method according to claim 1, wherein the root extract is a roots extract of *Helichrysum italicum.*

3. A process according to claim 1 or 2, wherein the extract is selected from an aqueous, oily, glycolic, organic or alcoholic extract of everlasting roots.

4. A process according to any one of the preceding claims in which the search for leontopodic acid is carried out by high performance liquid chromatography (HPLC), high performance liquid chromatography (HPLC) coupled with UV detection, by high performance liquid chromatography (HPLC) ) coupled to a mass spectrometer, by High Performance Liquid Chromatography (HPLC) coupled to a DEDL detector, by High Performance Liquid Chromatography (HPLC) coupled to a nuclear magnetic resonance device, by High Performance Liquid Chromatography (HPLC) coupled to an infra-red spectrometer, by Ultra High Performance Chromatography (UHPLC), by High Performance Thin Layer Chromatography (HPTLC), by High Performance Thin Layer Chromatography (HPTLC) coupled to a mass spectrometer, by Thin Layer Chromatography, by Supercritical Fluid Chromatography (SFC), by Supercritical Fluid Chromatography (SFC) coupled to a mass spectrometer, by Ionic Mobility, by Ionic Mobility Coupled to a mass spectrometer, by Capillary Electrophoresis, by Gas Chromatography (GC), by gas chromatography Coupled to a flame ionization detector, by gas chromatography coupled to a mass spectrometer, by Nuclear Magnetic Resonance, by infra-red spectrometry, by direct introduction mass spectrometry.

5. Use of leontopodic acid as a marker for the presence of an everlasting roots extract in a sample.

6. Use according to claim 5, wherein the extract is an oily extract of everlasting roots.

7. Use according to claim 5 wherein the extract is an aqueous extract of everlasting roots.

8. Use according to claim 5, wherein the extract is an alcoholic extract of everlasting roots.

9. Use according to claim 5, wherein the extract is a glycolic extract of everlasting roots.

10. Use according to claim 5, wherein the extract is an organic extract of everlasting roots

11. Use according to claim 5, wherein the sample is a sample of a cosmetic product.
